# EUROPEAN PATENT APPLICATION

(11) **EP 4 682 892 A1**
(43) Date of publication of application: **21.01.2026**
(21) Application number: 23927547.2
(22) Date of filing: 16.03.2023
(51) Int. Cl.: G16C 60/00

(54) **INFORMATION PROCESSING PROGRAM, INFORMATION PROCESSING METHOD, AND INFORMATION PROCESSING DEVICE**

(71) Applicant: FUJITSU LIMITED, Kawasaki-shi, Kanagawa 211-8588 (JP)
(72) Inventor: FUJITA, Kei, Kawasaki-shi, Kanagawa 211-8588 (JP); FUKUTA, Shigeki, Kawasaki-shi, Kanagawa 211-8588 (JP); HIGUCHI, Hiroyuki, Kawasaki-shi, Kanagawa 211-8588 (JP); ASAI, Tatsuya, Kawasaki-shi, Kanagawa 211-8588 (JP)
(74) Representative: Hoffmann Eitle
(86) International application number: PCT/JP2023/010472
(87) International publication number: WO 2024/189928

(57) **Abstract**

An information processing device (100) selects, as a unit of repetition, an entire space (711) of a three-dimensional model (700). In the selected space (711), the information processing device (100) detects that there are present, two parts (712, 713) of a pair having planar symmetry in the atomic arrangement and two parts (714, 715) of a pair having planar symmetry in the atomic arrangement, in an x-axis direction. The information processing device (100), exclusive of atoms included in one part (712, 715) of each detected pair, identifies, for example, one or more atoms at least partially included in the selected space (711). The information processing device (100) narrows down the range in which to search for the adsorption position of the adsorbate by excluding the atoms included in one part (712, 715) of each pair. The information processing device (100) determines the adsorption position of the adsorbate from among the identified one or more atoms for the three-dimensional model (700).

## Description

### TECHNICAL FIELD

Embodiments discussed herein relate to an information processing program, an information processing method, and an information processing device.

### BACKGROUND ART

Conventionally, there is a technology for performing a simulation in which an adsorbate is adsorbed on a catalyst of an alloy containing atoms of different metals, and analyzing the characteristics, etc. of the catalyst. For example, it is conceivable to comprehensively set combinations of the arrangement patterns of atoms of different metals in the catalyst and the adsorption positions of the adsorbate and perform a simulation.

As a prior art, for example, there is a technique of using density functional theory to propose heterogeneous catalysts based on datasets, by combining microkinetic analysis based on density functional theory with adversarial generative networks.

Non-Patent Literature 1: Ishikawa, Atsushi. "Heterogeneous catalyst design by generative adversarial network and first-principles based microkinetics." Scientific Reports 12.1 (2022): 11657.

### DISCLOSURE OF INVENTION

### PROBLEM TO BE SOLVED BY THE INVENTION

In the conventional technology, however, there is a problem that it is difficult to analyze the characteristics, etc. of a catalyst. For example, as the size of the catalyst increases, the number of combinations to be set also increases exponentially, and the processing time required to analyze the characteristics, etc. of the catalyst also increases.

In one aspect, the present invention aims to make it easier to analyze the characteristics, etc. of a catalyst.

### MEANS FOR SOLVING PROBLEM

According to one embodiment, an information processing program, an information processing method, and an information processing device are proposed that obtain a three-dimensional model representing an arrangement of multiple atoms that form a catalyst containing an atom of a certain metal; when the obtained three-dimensional model is a repetition of spaces in which a certain pattern of atomic arrangement appears in a first direction, select any of the spaces in which the certain pattern of atomic arrangement appears, from the obtained three-dimensional model; identify one or more atoms at least partially included in the selected any of the spaces; and determine, from among the identified one or more atoms of the obtained three-dimensional model, an atom to be replaced with an atom of another metal different from the certain metal, or an adsorption position of an adsorbate.

### EFFECT OF THE INVENTION

According to one aspect, it is possible to facilitate analysis of the characteristics, etc. of a catalyst.

### BRIEF DESCRIPTION OF DRAWINGS

Fig. 1 is an explanatory diagram depicting one example of an information processing method according to an embodiment.
Fig. 2 is an explanatory diagram depicting an example of an information processing system 200.
Fig. 3 is a block diagram of an example of a hardware configuration of the information processing device 100.
Fig. 4 is a block diagram depicting an example of a functional configuration of the information processing device 100.
Fig. 5 is an explanatory diagram depicting an example of operation of the information processing device 100.
Fig. 6 is an explanatory diagram depicting an example of operation of the information processing device 100.
Fig. 7 is an explanatory diagram depicting an example of operation of the information processing device 100.
Fig. 8 is an explanatory diagram depicting an example of operation of the information processing device 100.
Fig. 9 is an explanatory diagram depicting an example of operation of the information processing device 100.
Fig. 10 is a flowchart depicting an example of an overall processing procedure.

### BEST MODE(S) FOR CARRYING OUT THE INVENTION

An information processing program, an information processing method, and an information processing device according to an embodiment of the present disclosure are described in detail with reference to the accompanying drawings.

### (One Example of Information Processing Method According to Embodiment)

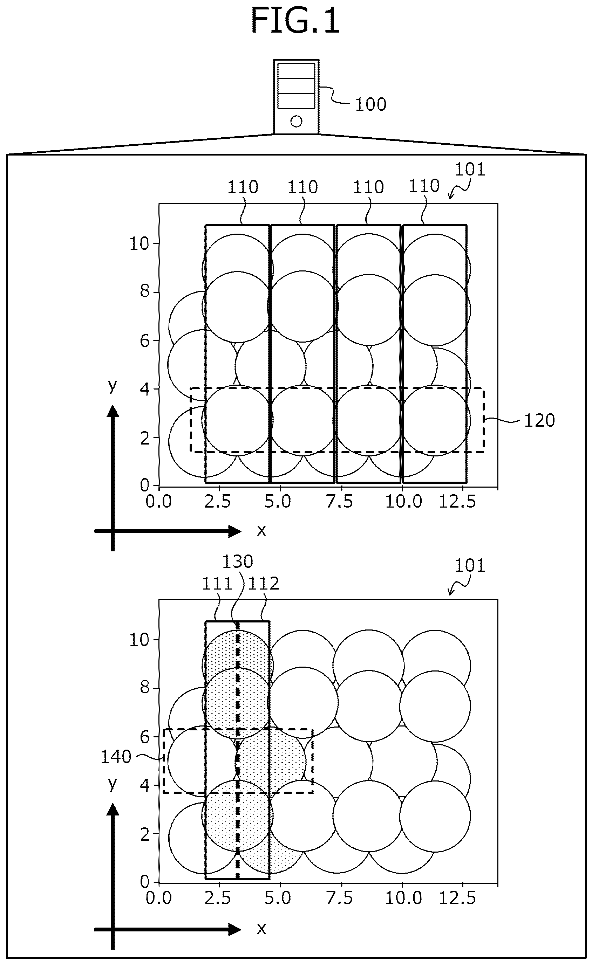

Fig. 1 is an explanatory diagram depicting one example of an information processing method according to an embodiment. An information processing device 100 is a computer for facilitating analysis of characteristics, etc. of a catalyst. The information processing device 100 is, for example, a server or a personal computer (PC).

Conventionally, it is desirable to analyze the characteristics, etc. of a catalyst. The catalyst-under-analysis is, for example, a binary alloy catalyst containing atoms of two kinds of metals. The catalyst-under-analysis may be, for example, an alloy catalyst containing atoms of three or more kinds of metals. For example, it is preferable to analyze the characteristics, etc. of the catalyst under various conditions related to the arrangement of atoms and the adsorption position of the adsorbent.

However, there is a problem that it is difficult to physically prepare, for each of the conditions related to the arrangement of atoms and the adsorption position of the adsorbent, a test catalyst that satisfies the condition. This results in a problem that it is difficult to analyze the characteristics, etc. of a catalyst under various conditions related to the arrangement of atoms and the adsorption position of the adsorbent.

It is therefore desirable to analyze the characteristics, etc. of a catalyst by performing on a computer, a simulation of the reaction or action of the adsorbent on the catalyst under various conditions related to the arrangement of atoms and the adsorption position of the adsorbent.

For example, a technique 1 is conceivable in which combinations of the arrangement patterns of different metal atoms in a catalyst and the adsorption positions of adsorbents are comprehensively set so that a simulation is performed for each of the set combinations.

Even with this technique 1, there remains a problem that it is difficult to analyze the characteristics, etc. of the catalyst. For example, as the size of the catalyst increases, the number of combinations to be set increases exponentially and the processing time required to perform a simulation also increases.

In contrast, a technique 2 is conceivable in which combinations of the arrangement patterns of different metal atoms in a catalyst and the adsorption positions of adsorbents are randomly set so that a simulation is performed for each of the set combinations.

Even with this technique 2 a problem still remains in that it is difficult to analyze the characteristics, etc. of the catalyst. For example, there is an issue that the combinations to be set may overlap, making it difficult to accurately analyze the characteristics, etc. of the catalyst. For example, in each of two set combinations, the positional relationship between the atoms of each metal and the adsorbents may be substantially the same, resulting in a case in which an unnecessary simulation may be carried out.

On the other hand, a technique 3 may be considered in which, for example, multiple combinations of arrangement patterns of atoms of different metals in a catalyst and adsorption positions of adsorbates are set without overlap so that a simulation is performed for each of the set combinations.

Even with this technique 3, a problem still remains in that it is difficult to analyze the characteristics, etc. of the catalyst. For example, the set combinations may be artificial, making it difficult to accurately analyze the characteristics, etc. of the catalyst.

Thus, in the present embodiment, an information processing method will be described that may facilitate the analysis of the characteristics, etc. of a catalyst.

In Fig. 1, the information processing device 100 obtains a three-dimensional model 101 that represents the arrangement of multiple atoms that form a catalyst containing atoms of a certain metal. The certain metal is, for example, a base metal that forms an alloy. It is assumed that the catalyst contains only atoms of a certain metal in the initial state. The three-dimensional model 101 represents, for example, the arrangement of multiple atoms of a certain metal that form a catalyst in a three-dimensional space in the initial state.

In the following description, the information processing device 100 processes the three-dimensional model 101 so as to represent a sample of the state of an alloy catalyst suitable for a simulation. For example, the information processing device 100 searches for an atom to be replaced with an atom of another metal different from a certain metal among the multiple atoms forming the three-dimensional model 101 so as to represent an alloy catalyst. This makes it easier for the information processing device 100 to prepare a sample of the state of an alloy catalyst suitable for a simulation.

(1-1) The information processing device 100 determines whether the obtained three-dimensional model 101 is a repetition of a space in which a certain pattern of atomic arrangement appears in a first direction. The first direction is an axial direction of the three-dimensional space. The first direction is, for example, the x-axis direction. In the example of Fig. 1, the information processing device 100 determines that the three-dimensional model 101 is a repetition of a space 110 in which a certain pattern of atomic arrangement appears in the x-axis direction.

Here, since the same pattern of atomic arrangement appears in each space 110, it is considered equivalent to replace each atom present at the same position in different spaces 110 with an atom of another metal different from the certain metal. In the example of Fig. 1, for example, it is considered equivalent to replace each atom present in a range 120, with an atom of another metal different from the certain metal. Hence, when searching for an atom to be replaced, it is considered possible to substantially consider all of the spaces 110 even when only one of the spaces 110 is focused on.

(1-2) When the three-dimensional model 101 is a repetition of spaces in which a certain pattern of atomic arrangement appears in the first direction, the information processing device 100 selects one of the spaces in which the certain pattern of atomic arrangement appears from the three-dimensional model 101. In the example of Fig. 1, for example, the information processing device 100 selects one of the spaces 110 from the three-dimensional model 101. Thereby, the information processing device 100 may narrow down to one of the spaces 110, the range in which the atom to be replaced is searched for. Therefore, the information processing device 100 may reduce the processing time required to search for an atom to be replaced.

(1-3) The information processing device 100 determines whether in any of the selected spaces, there are two parts having symmetry in the arrangement of atoms in the first direction. Symmetry is, for example, planar symmetry. In the example of Fig. 1, for example, the information processing device 100 determines that there are two parts 111 and 112 having planar symmetry with respect to a symmetry plane 130 perpendicular to the first direction in any of the spaces 110.

Here, since the parts 111 and 112 have planar symmetry, it is considered that replacing each atom present at a planar symmetrical position in each of the parts 111 and 112 with an atom of a metal other than the certain metal is equivalent. In the example of Fig. 1, for example, it is considered that replacing each atom present in a range 140 with an atom of a metal other than the certain metal is equivalent. Therefore, when searching for an atom to be replaced, it is considered that even when only one of the two parts 111 and 112 is focused on, the other may be substantially considered.

(1-4) When there are two parts having symmetry in the arrangement of atoms, the information processing device 100, exclusive of the atoms included in one of the two parts, identifies one or more atoms of the three-dimensional model 101, at least a part of which is included in any of the selected spaces. The information processing device 100, exclusive of the atoms completely contained in one of the parts, identifies, for example, one or more atoms that at least a part of which is included in any of the selected spaces.

In the example of Fig. 1, the information processing device 100, exclusive of the atoms included in the part 111, for example, identifies multiple hatched atoms at least a part of which is included in any of the selected spaces 110. This allows the information processing device 100 to narrow down the range in which to search for an atom to be replaced, to one or more atoms. Therefore, the information processing device 100 may reduce the processing time required to search for an atom to be replaced.

(1-5) The information processing device 100 determines an atom to be replaced with an atom of a metal different from the one metal among the one or more specified atoms for the three-dimensional model 101. For example, the information processing device 100 randomly determines an atom to be replaced with an atom of a metal different from the one metal among the one or more specified atoms for the three-dimensional model 101.

As described, the information processing device 100 may process the three-dimensional model 101 to represent a sample of the state of the alloy catalyst suitable for simulation. The information processing device 100 may easily prepare a sample of the state of the alloy catalyst suitable for simulation.

The information processing device 100 may easily create simulation data in which, for example, a sample of the state of the alloy catalyst is associated with the adsorption position of the adsorbent, and may easily analyze the characteristics, etc. of the catalyst. The adsorption position is, for example, a contact position in the three-dimensional model 101 that contacts all three atoms that are adjacent to each other.

The information processing device 100 may reduce the number of samples of the state of the alloy catalyst used in the simulation. Therefore, the information processing device 100 may reduce the processing time required to perform a simulation and analyze the characteristics, etc. of the catalyst.

The information processing device 100 may easily prepare samples of the state of the alloy catalyst used in the simulation without duplication. Therefore, the information processing device 100 may easily perform a simulation and analyze the characteristics, etc. of the catalyst with high accuracy.

The information processing device 100 may easily prepare random samples of the state of the alloy catalyst used in the simulation. Therefore, the information processing device 100 may easily avoid the samples from being artificial, and may easily analyze the characteristics, etc. of the catalyst with high accuracy.

(1-6) The information processing device 100 creates simulation data in which the samples of the state of the alloy catalyst correspond to the adsorption position of the adsorbate. The information processing device 100 performs a simulation regarding the reaction or action of the adsorbate to the catalyst based on the simulation data. This allows the information processing device 100 to analyze the characteristics, etc. of the catalyst.

Here, for the sake of simplicity, a case where the information processing device 100 determines the atom to be replaced is described, and a case where the adsorption position of the adsorbent is determined is omitted. The information processing device 100 may determine the adsorption position of the adsorbent in the same manner as in the case where the atom to be replaced is determined.

For example, as in the case where the atom to be replaced is determined, the three-dimensional model 101 after the replacement may be a repetition of spaces in which a certain pattern of atomic arrangement appears in the first direction. In this case, as in the case where the atom to be replaced is determined, it is considered equivalent to determine the same position in different spaces as the adsorption position of the adsorbent. Therefore, as in the case where the atom to be replaced is determined, the information processing device 100 may narrow down the range for searching for the adsorption position of the adsorbent, and may reduce the processing time required to determine the adsorption position of the adsorbent.

For example, as in the case where the atom to be replaced is determined, it is considered that there are two parts having symmetry in the atomic arrangement in the first direction in any of the repeated spaces in the three-dimensional model 101 after the replacement. In this case, it is considered equivalent to determine the plane-symmetrical positions in each part as the adsorption positions of the adsorbate, as in the case of determining the atom to be replaced. Therefore, the information processing device 100 may narrow down the range for searching the adsorption positions of the adsorbate, as in the case of determining the atom to be replaced, and may reduce the processing time required to determine the adsorption positions of the adsorbate.

This allows the information processing device 100 to appropriately determine the adsorption positions of the adsorbate. The information processing device 100 may, for example, determine the adsorption positions of the adsorbate without overlapping. The information processing device 100 may, for example, randomly determine the adsorption positions of the adsorbate. The information processing device 100 may, for example, easily create simulation data in which a sample of the state of the alloy catalyst is associated with the adsorption positions of the adsorbate, and may easily analyze the characteristics, etc. of the catalyst with high accuracy.

Here, while a case where the information processing device 100 considers both the repetition of the space in which the atomic arrangement of the same pattern appears in the three-dimensional model 101 and the symmetry of the atomic arrangement appearing in two parts of the three-dimensional model 101 has been described, the present invention is not limited thereto. There may be a case where the information processing device 100 considers only one of the repetition of the space in which the atomic arrangement of the same pattern appears in the three-dimensional model 101 and the symmetry of the atomic arrangement appearing in two parts of the three-dimensional model 101.

For example, after selecting one of the spaces in the three-dimensional model 101, the information processing device 100 may specify one or more atoms at least a part of which is included in the one of the spaces without considering the symmetry of the atomic arrangement. Then, for example, the information processing device 100 may determine an atom to be replaced with an atom of another metal different from a certain metal from among the one or more specified atoms in the three-dimensional model 101. Thereby, the information processing device 100 may narrow down the range in which the atom to be replaced is searched, and the processing time required for searching the atom to be replaced may be reduced.

The information processing device 100 may determine whether there are two parts having symmetry in the arrangement of atoms in the first direction in the entire space of the three-dimensional model 101, without considering, for example, the repetition of the space in which the arrangement of atoms of the same pattern appears. Next, the information processing device 100 may specify one or more atoms of the three-dimensional model 101, the atoms being at least partially included in the entire space of the three-dimensional model 101, other than the atoms included in one of the two parts. Then, the information processing device 100 may determine, for example, an atom to be replaced with an atom of another metal different from the certain metal, from among the one or more specified atoms of the three-dimensional model 101. Thereby, the information processing device 100 may narrow down the range in which the atom to be replaced is searched, and the processing time required for searching the atom to be replaced may be reduced.

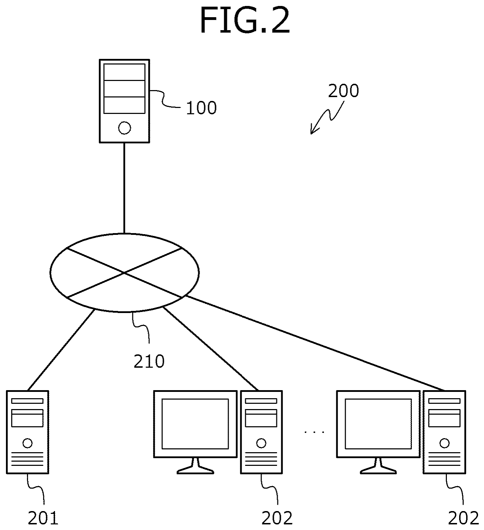

Here, the case where the information processing device 100 operates independently has been described, but this is not limitative. For example, the information processing device 100 may cooperate with another computer. For example, the information processing device 100 may cooperate with another computer capable of performing a simulation. For example, a plurality of computers may cooperate to realize the functions of the information processing device 100 described above. For example, the functions of the information processing device 100 may be realized on the cloud. A case in which the information processing device 100 cooperates with another computer will be described later with reference to Fig. 2.

### (An Example of Information Processing System 200)

Next, with reference to Fig. 2, an example of an information processing system 200 to which the information processing device 100 depicted in Fig. 1 is applied will be described.

Fig. 2 is an explanatory diagram depicting an example of the information processing system 200. In Fig. 2, the information processing system 200 includes the information processing device 100, a simulation device 201, and a client device 202.

In the information processing system 200, the information processing device 100 and the simulation device 201 are coupled via a wired or wireless network 210. The network 210 is, for example, a local area network (LAN), a wide area network (WAN), the Internet, or the like. In the information processing system 200, the information processing device 100 and the client device 202 are coupled via the wired or wireless network 210.

The information processing device 100 is a computer for facilitating analysis of the characteristics, etc. of a catalyst. The information processing device 100 receives a processing request from the client device 202. The processing request includes, for example, a parameter for specifying the shape of the catalyst. The parameter indicates, for example, the surface shape of the catalyst. The parameter indicates, for example, two or more metals that form an alloy of the catalyst.

The information processing device 100 specifies, based on the processing request, two or more metals that form an alloy of the catalyst. The information processing device 100 sets one of the specified two or more metals as a base metal, and sets the other metal as a replacement metal. The information processing device 100 specifies, based on the processing request, the shape of the catalyst. The information processing device 100 generates a three-dimensional model representing the arrangement of multiple atoms of the base metal forming the catalyst in the case where the catalyst is formed only from the atoms of the set base metal according to the specified shape.

The information processing device 100 determines atoms to replace the atoms of the set replacement metal in the generated three-dimensional model, as in Fig. 1. The information processing device 100 replaces the determined atoms in the generated three-dimensional model with the atoms of the set replacement metal. The information processing device 100 determines the adsorption position of the adsorbate in the three-dimensional model after the replacement, as in Fig. 1. The information processing device 100 generates simulation data in which the three-dimensional model after the replacement and the determined adsorption position of the adsorbate are associated with each other.

The information processing device 100 transmits the generated simulation data to the simulation device 201. The information processing device 100 receives the simulation result from the simulation device 201. The information processing device 100 transmits the received simulation result to the client device 202. The information processing device 100 is, for example, a server or a PC.

The simulation device 201 is a computer for performing a simulation regarding the reaction or action of an adsorbate on a catalyst. The simulation device 201 receives, for example, simulation data from the information processing device 100. The simulation device 201 performs, for example, a simulation regarding the reaction or action of an adsorbate on a catalyst. The simulation device 201 transmits the simulation result to the information processing device 100. The simulation device 201 is, for example, a server or a PC.

The client device 202 is a computer used by an operator who is trying to grasp the characteristics, etc. of a catalyst. The client device 202 generates a processing request based on the operator's operation input and transmits it to the information processing device 100. The processing request includes, for example, a parameter that specifies the shape of the catalyst. The parameter indicates, for example, the surface shape of the catalyst. The parameter indicates, for example, two or more metals that form an alloy of the catalyst.

The client device 202 receives the simulation result from the information processing device 100. The client device 202 outputs the simulation result so that the operator may understand it. The client device 202 is, for example, a PC, a tablet terminal, or a smartphone.

Here, the case where the information processing device 100 is a device different from the simulation device 201 has been described, but this is not limited thereto. For example, the information processing device 100 may have a function as the simulation device 201 and operate as the simulation device 201.

Here, the case where the information processing device 100 is a device different from the client device 202 has been described, but this is not limited thereto. For example, the information processing device 100 may have a function as the client device 202 and operate as the client device 202.

Here, the case where the simulation device 201 is a device different from the client device 202 has been described, but this is not limited thereto. For example, the simulation device 201 may have a function as the client device 202 and operate as the client device 202.

### (Example of Hardware Configuration of Information Processing Device)

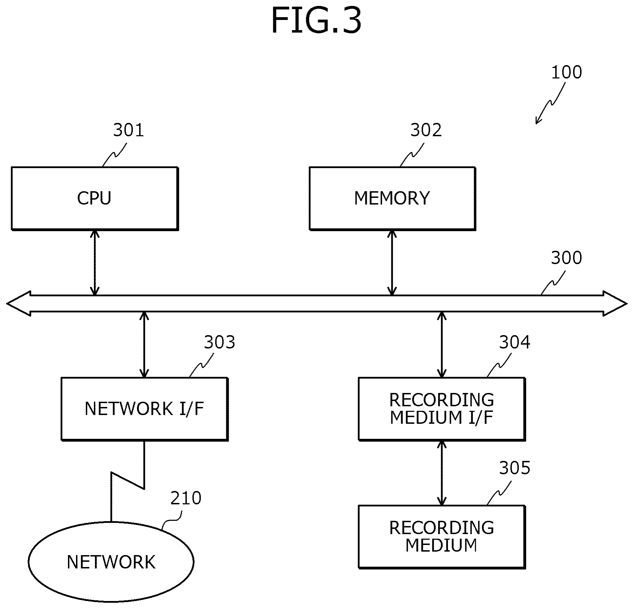

Next, with reference to Fig. 3, an example of hardware configuration of the information processing device 100 is described.

Fig. 3 is a block diagram of an example of a hardware configuration of the information processing device 100. In Fig. 3, the information processing device 100 has a central processing unit (CPU) 301, a memory 302, a network interface (I/F) 303, a recording medium I/F 304, and recording medium 305. Further, the components are coupled to each other by a bus 300.

The CPU 301 governs overall control of the information processing device 100. The memory 302, for example, includes a read-only memory (ROM), a random-access memory (RAM) and a flash ROM. In particular, for example, the flash ROM and the ROM store various types of pf programs and the RAM is used as a work area of the CPU 301. Programs stored in the memory 302 are loaded onto the CPU 301, whereby encoded processes are executed by the CPU 301.

The network I/F 303 is coupled to the network 210 through a communications line and is coupled to other computers via the network 210. Further, the network I/F 303 administers an internal interface with the network 210 and controls the input and output of data with respect to other computers. The network I/F 303, for example, is a modem or a LAN adapter.

The recording medium I/F 304, under the control of the CPU 301, controls the reading and writing of data with respect to the recording medium 305. The recording medium I/F 304 is, for example, a disk drive, a solid-state drive (SSD), a universal serial bus (USB) port, or the like. The recording medium 305 is a nonvolatile memory storing therein data written thereto under the control of the recording medium I/F 304. The recording medium 305 is, for example, a disk, a semiconductor memory, a USB memory, or the like. The recording medium 305 may be removable from the information processing device 100.

The information processing device 100, in addition to the described components, may further have, for example, a camera or the like. Further, in addition to the components above, the information processing device 100 may further have, for example, a keyboard, a mouse, a display, a printer, a scanner, a microphone, or a speaker. Further, the information processing device 100, for example, may omit the recording medium I/F 304 and the recording medium 305.

### (Example of Hardware Configuration of Simulation Device 201)

The hardware configuration of the simulation device 201 is, for example, similar to the hardware configuration of the information processing device 100 depicted in Fig. 3 and therefore, description thereof is omitted.

### (Example of Hardware Configuration of Client Device 202)

The hardware configuration of the client device 202 is, for example, similar to the hardware configuration of the information processing device 100 depicted in Fig. 3 and therefore, a description thereof is omitted.

In the following description, a case will be mainly described where the information processing apparatus 100 operates independently.

### (Example of Functional Configuration of Information Processing Device 100)

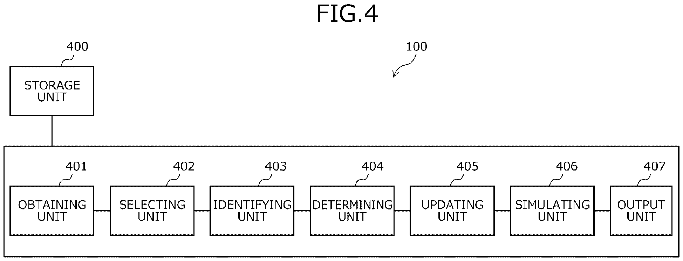

An example of a functional configuration of the information processing device 100 will then be described with reference to Fig. 4.

Fig. 4 is a block diagram depicting an example of the functional configuration of the information processing device 100. The information processing device 100 includes a storage unit 400, an obtaining unit 401, a selecting unit 402, an identifying unit 403, a determining unit 404, an updating unit 405, a simulating unit 406, and an output unit 407.

The storage unit 400 is realized, for example, by a storage area such as the memory 302 or the recording medium 305 depicted in Fig. 3. In the following, a case where the storage unit 400 is included in the information processing device 100 will be described, but this is not limitative. For example, the storage unit 400 may be included in a device different from the information processing device 100, and the stored contents of the storage unit 400 may be referred to from the information processing device 100.

The obtaining unit 401 to the output unit 407 function as an example of a controller. For example, functions the obtaining unit 401 to the output unit 407 are realized by, for example, causing the CPU to execute a program stored in a storage area such as the memory 302 or the recording medium 305 depicted in Fig. 3, or by the network I/F 303. The processing results of each functional unit are stored in a storage area such as the memory 302 or the recording medium 305 depicted in Fig. 3.

The storage unit 400 stores various types of information that is referred to or updated in the processing by each functional unit. The storage unit 400 stores, for example, parameters that specify the shape of the catalyst. The parameters indicate, for example, the surface shape of the catalyst. The parameters indicate, for example, two or more metals that form an alloy of the catalyst. The two or more metals include, for example, one base metal and one or more replacement metals. The parameters are obtained by, for example, the obtaining unit 401.

The storage unit 400 stores, for example, a three-dimensional model that indicates the arrangement of multiple atoms that form the catalyst. The three-dimensional model represents the arrangement of a plurality of atoms of a base metal forming the catalyst in the initial state, assuming that the catalyst is formed only of the atoms of the base metal. The three-dimensional model is obtained by, for example, the obtaining unit 401. The three-dimensional model is updated by, for example, the updating unit 405.

The obtaining unit 401 obtains various types of information used in the processing by each functional unit. The obtaining unit 401 stores the obtained various types of information in the storage unit 400, or outputs it to each functional unit. The obtaining unit 401 may also output the various types of information stored in the storage unit 400 to each functional unit. The obtaining unit 401 obtains various types of information, for example, based on an operation input by a user. The obtaining unit 401 may receive various types of information, for example, from a device different from the information processing device 100.

The obtaining unit 401 obtains, for example, parameters specifying the shape of the catalyst. For example, the obtaining unit 401 obtains parameters specifying the shape of the catalyst by receiving them from another computer. The obtaining unit 401 may obtain parameters for specifying the shape of the catalyst by receiving input of parameters for specifying the shape of the catalyst based on an operation input by a user.

The obtaining unit 401 may obtain, for example, a three-dimensional model representing the arrangement of a plurality of atoms forming the catalyst. The obtaining unit 401 may obtain, for example, a three-dimensional model representing the arrangement of a plurality of atoms of a base metal forming the catalyst in a case where the catalyst is formed only from atoms of the base metal according to the specified shape. The obtaining unit 401 may obtain, for example, a three-dimensional model by receiving the three-dimensional model from another computer. The obtaining unit 401 may obtain, for example, a three-dimensional model by receiving input of the three-dimensional model based on an operation input by a user.

The obtaining unit 401 may accept a start trigger for starting processing by any of the functional units. The start trigger may be, for example, a predetermined operation input by a user. The start trigger may be, for example, a reception of predetermined information from another computer. The start trigger may be, for example, the output of predetermined information by any of the functional units. For example, the obtaining unit 401 may receive the acquisition of a three-dimensional model as a start trigger for starting the processing by the selecting unit 402, the identifying unit 403, and the determining unit 404.

The selecting unit 402 selects a space included in the three-dimensional model obtained by the obtaining unit 401 or updated by the updating unit 405. The selecting unit 402 determines, for example, whether the three-dimensional model is a repetition of spaces in which a certain pattern of atomic arrangement appears in a first direction. The pattern of atomic arrangement is a pattern including the adsorption position of the adsorbent, for example, if the adsorption position of the adsorbent for the three-dimensional model representing the catalyst has been determined by the determining unit 404.

When the three-dimensional model is a repetition of spaces in which a certain pattern of atomic arrangement appears in a first direction, the selecting unit 402 selects any space in the three-dimensional model in which the certain pattern of atomic arrangement appears. This allows the selecting unit 402 to narrow down the range in which the atoms to be replaced by the atoms of the replacement metal or the adsorption position of the adsorbate are searched for.

When the three-dimensional model is not a repetition of a space in which a certain pattern of atomic arrangement appears in the first direction, the selecting unit 402 selects the entire space of the three-dimensional model. This allows the selecting unit 402 to appropriately set the range in which the atoms to be replaced by the atoms of the replacement metal or the adsorption position of the adsorbate are searched for.

The selecting unit 402 may select any one of the spaces by accepting a designation of any one of the spaces in the three-dimensional model based on, for example, an operation input by a user.

The identifying unit 403 identifies one or more atoms at least a part of which is included in the space selected by the selecting unit 402 from the three-dimensional model obtained by the obtaining unit 401 or updated by the updating unit 405. The identifying unit 403 determines, for example, whether two parts having symmetry in the atomic arrangement exist in the first direction in the space selected by the selecting unit 402. The symmetry is, for example, symmetry including the adsorption position of the adsorbate in the three-dimensional model representing the catalyst, if the determining unit 404 has already determined the adsorption position of the adsorbate.

When, for example, two parts exist, the identifying unit 403 identifies one or more atoms of the three-dimensional model, at least a part of which is included in the space selected by the selecting unit 402, other than the atoms included in one of the two parts. This allows the identifying unit 403 to narrow down the range in which the atoms to be replaced by the atoms of the replacement metal, or the adsorption position of the adsorbate are searched for. The identifying unit 403 may appropriately identify one or more atoms as the atoms to be replaced by the atoms of the replacement metal, or the range in which the adsorption position of the adsorbate is searched for.

When, for example, two parts do not exist, the identifying unit 403 identifies one or more atoms of the three-dimensional model, at least a part of which is included in the space selected by the selecting unit 402. This allows the identifying unit 403 to appropriately identify one or more atoms as the atoms to be replaced by the atoms of the replacement metal, or the range in which the adsorption position of the adsorbate is searched for.

The identifying unit 403 may identify two parts by accepting designation of two parts having symmetry in the arrangement of atoms in a first direction in the space selected by the selecting unit 402, for example, based on an operational input by a user.

The determining unit 404 determines an atom to be replaced with an atom of the replacement metal or an adsorption position of an adsorbate from among one or more atoms identified by the identifying unit 403 in the three-dimensional model obtained by the obtaining unit 401 or updated by the updating unit 405. The determining unit 404 randomly determines an atom to be replaced with an atom of the replacement metal from among one or more atoms identified by the identifying unit 403 in the three-dimensional model, for example. As described, the determining unit 404 may update the three-dimensional model to represent a sample of the state of the alloy catalyst suitable for simulation.

The determining unit 404 determines, for example, a contact position in the three-dimensional model where all three of the atoms identified by the identifying unit 403 are in contact with each other, as the adsorption position of the adsorbate. This enables the determining unit 404 to generate simulation data in which a sample of the state of the alloy catalyst is associated with the adsorption position of the adsorbate.

The updating unit 405 updates the three-dimensional model. For example, when the updating unit 405 determines, from among the one or more identified atoms, an atom to be replaced with an atom of a replacement metal different from the base metal, the updating unit 405 replaces the atom in the three-dimensional model with an atom of a replacement metal different from the base metal. This enables the updating unit 405 to update the three-dimensional model to represent a sample of the state of the alloy catalyst suitable for the simulation.

The updating unit 405 generates simulation data. For example, the updating unit 405 generates simulation data in which the updated three-dimensional model is associated with the adsorption position of the adsorbate determined by the determining unit 404. This allows the updating unit 405 to perform a simulation.

The simulating unit 406 performs a simulation based on the simulation data generated by the updating unit 405. For example, the simulating unit 406 performs a simulation regarding the reaction or action of the adsorbent on the catalyst when the adsorbent is adsorbed on the catalyst based on the simulation data generated by the updating unit 405. This allows the simulating unit 406 to perform a simulation and analyze the characteristics, etc. of the catalyst.

The output unit 407 outputs the processing result of at least one of the functional units. The output format is, for example, display on a display, print output to a printer, transmission to an external device via the network I/F 303, or storage in a storage area such as the memory 302 or the recording medium 305. This allows the output unit 407 to notify the user of the processing result of at least one of the functional units, thereby improving the convenience of the information processing device 100.

The output unit 407 outputs, for example, a three-dimensional model updated by the updating unit 405. The output unit 407 for example outputs the three-dimensional model so that the user may refer to it. The output unit 407 for example may transmit the three-dimensional model to another computer. As described, the output unit 407 may make the three-dimensional model available externally. The output unit 407 may facilitate the generation of simulation data externally.

The output unit 407 outputs, for example, the simulation data generated by the updating unit 405. For example, the output unit 407 outputs the simulation data so that the user may refer to it. For example, the output unit 407 may transmit the simulation data to another computer. As described, the output unit 407 may make it possible to perform a simulation externally.

The output unit 407 outputs, for example, the result of the simulation performed by the simulating unit 406. For example, the output unit 407 outputs the result of the simulation so that the user may refer to it. For example, the output unit 407 may transmit the result of the simulation to another computer. As described, the output unit 407 may make the result of the simulation available externally.

Here, the case has been described where the selecting unit 402 determines whether the three-dimensional model is a repetition of a space in which a certain pattern of atomic arrangement appears in the first direction, but this is not limitative. For example, there may be a case in which the selecting unit 402 does not determine whether the three-dimensional model is a repetition of a space in which a certain pattern of atomic arrangement appears in the first direction.

Here, the case has been described where the identifying unit 403 determines whether there are two parts having symmetry in the arrangement of atoms in the first direction in the selected space, but the present invention is not limited thereto. For example, the identifying unit 403 may not determine whether there are two parts having symmetry in the arrangement of atoms in the first direction in the selected space.

Here, the case has been described where the information processing device 100 includes the obtaining unit 401, the selecting unit 402, the identifying unit 403, the determining unit 404, the updating unit 405, the simulating unit 406, and the output unit 407, but the present invention is not limited thereto.

For example, the information processing device 100 may omit any of the functions. For example, the other computer may include any of the functions. For example, the other computer may include the simulating unit 406. In this case, the information processing device 100 utilizes any of the functional units by working with the other computer.

### (Example of Operation of Information Processing Device 100)

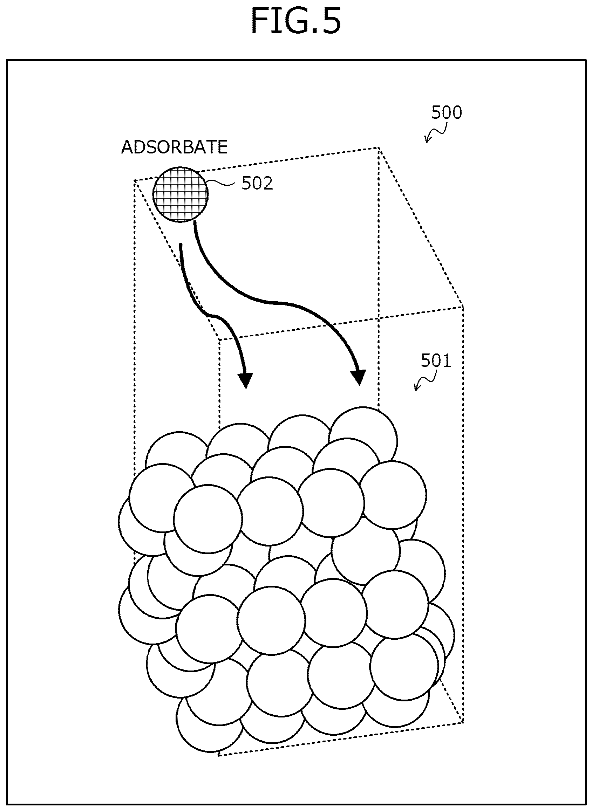

An example of operation of the information processing device 100 will then be described with reference to Figs. 5 to 9.

Figs. 5, 6, 7, 8, and 9 are explanatory diagrams depicting an example of operation of the information processing device 100. In Fig. 5, it is assumed that it is desired to carry out a simulation of the reaction or action of an adsorbate 502 on a catalyst 501 represented by a three-dimensional model 500 when the adsorbate 502 is adsorbed on the catalyst 501.

The information processing device 100, for example, receives a processing request including parameters specifying the shape of the catalyst 501 and requesting the carrying out of a simulation. The parameters indicate, for example, the surface shape of the catalyst 501. The parameters indicate, for example, two or more metals that form an alloy of the catalyst 501. The system of the catalyst 501 is assumed to be under periodic boundary conditions. For example, the system of the catalyst 501 is assumed to be under periodic boundary conditions in the x-axis direction and the y-axis direction.

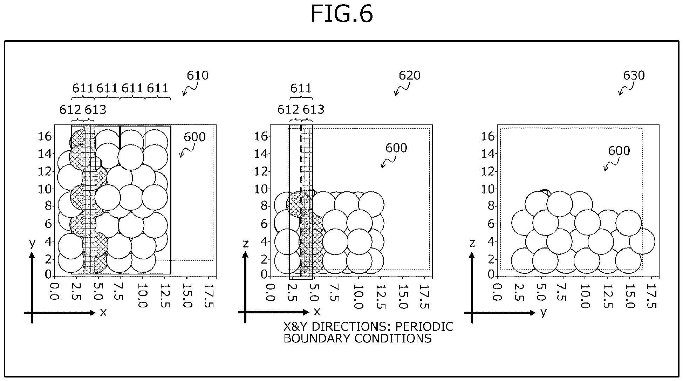

Hence, the information processing device 100 determines whether it is preferable to perform a simulation in terms of how atoms of different metals are arranged in the catalyst 501 and how the adsorbate is adsorbed, as indicated below, and generates simulation data. Next, we move on to the explanation of Fig. 6.

In Fig. 6, the information processing device 100 specifies a step structure as the surface structure of the catalyst 501 based on the processing request. The surface structure may be a kink, an adatom, or a vacancy. In the step structure, the adsorbate adsorption position is limited to, for example, the upper stage of the catalyst 501. The information processing device 100 specifies two or more metals that form an alloy of the catalyst 501 based on the processing request. The information processing device 100 sets one of the two or more metals as a base metal, and sets each of the other metals as a replacement metal.

For example, two or more replacement metals may exist. In the following explanation, a case in which there are two replacement metals will be explained. In addition, the first replacement metal may be written as the "first replacement metal" and the second replacement metal may be written as the "second replacement metal".

The information processing device 100 generates a three-dimensional model 600 corresponding to the catalyst 501 formed only with atoms of the set base metal. The three-dimensional model 600 represents the arrangement of a plurality of atoms in the catalyst 501. A graph 610 depicts the three-dimensional model 600 from the z-axis direction. A graph 620 depicts the three-dimensional model 600 from the y-axis direction. A graph 630 depicts the three-dimensional model 600 from the x-axis direction.

(6-1) When the three-dimensional model 600 is a repetition of spaces in which a certain pattern of atomic arrangement appears in the x-axis direction, the information processing device 100 selects one of spaces 611 that is a repeating unit. Here, it is considered that replacing atoms present at the same position in different spaces 611 with atoms of the first replacement metal is equivalent. Therefore, by selecting any one of the spaces 611, the information processing device 100 may narrow down the range in which the base metal atoms to replace the first replacement metal atoms are searched for.

(6-2) The information processing device 100 specifies one or more atoms at least a part of which is included in any one of the selected spaces 611, and specifies the range in which the base metal atoms to replace the first replacement metal atoms are searched for. For example, the information processing device 100 detects that, in any one of the selected spaces 611, there are two parts 612 and 613 having plane symmetry in the arrangement of atoms in the x-axis direction. For example, the information processing device 100 specifies one or more atoms at least a part of which is included in any one of the selected spaces 611 other than the atoms included in one part 612.

In the example of Fig. 6, the information processing device 100 for example specifies the atoms indicated by cross hatching. Here, it is considered that replacing each atom of the base metal present in a plane symmetrical position in each of the parts 612 and 613 with an atom of the first replacement metal is equivalent. Therefore, the information processing device 100 may narrow down the range of searching for the base metal atom to replace the first replacement metal atom by excluding the atom included in one part 612.

(6-3) The information processing device 100 determines the base metal atom to replace the first replacement metal atom from among the one or more specified atoms for the three-dimensional model 600. For example, the information processing device 100 randomly determines the base metal atom to replace the first replacement metal atom from among the one or more specified atoms for the three-dimensional model 600.

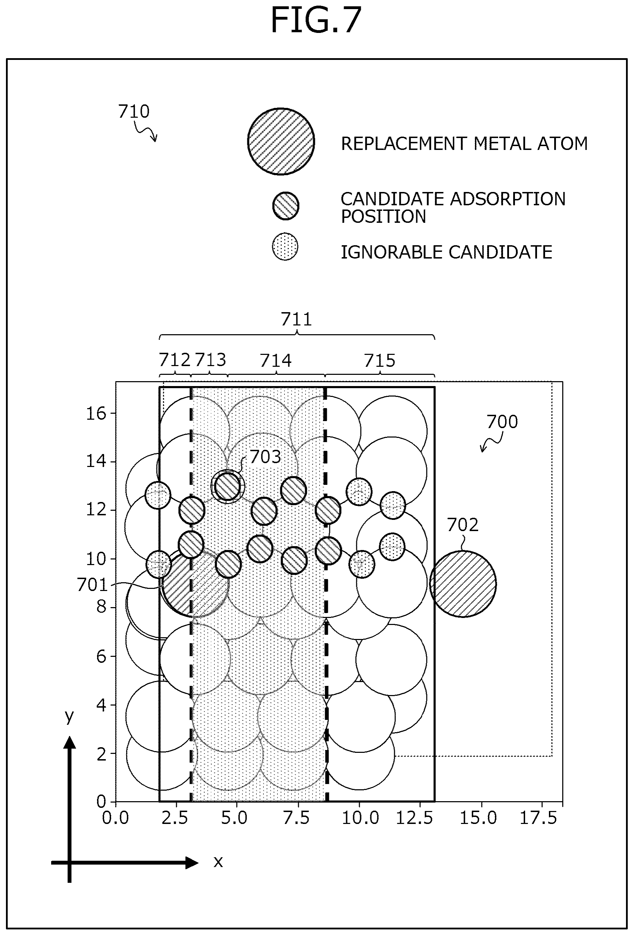

The information processing device 100 updates the three-dimensional model 600 to a three-dimensional model 700 that will be described later in Fig. 7 so that the determined base metal atom replaces the first replacement metal atom. Here, in order to improve the accuracy of analyzing the characteristics, etc. of the catalyst, etc. and to reduce the artificiality of the updated three-dimensional model 700, it may be preferable for the information processing device 100 to randomly determine the base metal atom to replace the first replacement metal atom.

As a result, the information processing device 100 may determine the base metal atoms to be replaced by the first replacement metal atoms in the three-dimensional model 600, taking into consideration the overlap of the positions to be replaced by the atoms of the first replacement metal. The information processing device 100 may reduce the range of searching for the base metal atoms to be replaced by the atoms of the first replacement metal to about 1/4. Therefore, the information processing device 100 may update the three-dimensional model 700 after updating so that it represents a catalyst suitable for a simulation including atoms of different metals.

Here, the case where the information processing device 100 randomly determines the base metal atoms to be replaced by the first replacement metal atoms from among one or more specified atoms for the three-dimensional model 600 has been described, but is not limited thereto. For example, the information processing device 100 may comprehensively determine the base metal atoms to be replaced by the first replacement metal atoms from among one or more specified atoms for the three-dimensional model 600.

The information processing device 100 may prepare N updated three-dimensional models 700 by updating the three-dimensional model 600 so that each atom of the determined base metal is replaced with an atom of the first replacement metal. N is the number of atoms specified, which is 1 or more. In this case, the information processing device 100 executes the following process for each updated three-dimensional model 700. Next, we move on to the explanation of Fig. 7.

In Fig. 7, a graph 710 depicts the updated three-dimensional model 700 from the z-axis direction. In the three-dimensional model 700, an atom 701 of the first replacement metal, indicated by hatching with diagonal lines slanting upward to the right, exists. According to the periodic boundary condition, the information processing device 100 may consider that an atom 702 of the first replacement metal, indicated by hatching with diagonal lines slanting upward to the right, exists in the three-dimensional model 700 due to the existence of the atom 701 of the first replacement metal.

(7-1) The information processing device 100 determines that the three-dimensional model 700 is not a repetition of a space in which a certain pattern of atomic arrangement appears in the x-axis direction. Hence, the information processing device 100 selects an entire space 711 of the three-dimensional model 700 as a repetition unit.

(7-2) The information processing device 100 specifies one or more atoms at least a part of which is included in the selected space 711, and specifies a range in which to search for the adsorption position of the adsorbate. For example, the information processing device 100 detects that, in the selected space 711, there are two parts 712 and 713 in a pair having plane symmetry in the atomic arrangement, and two parts 714 and 715 in a pair having plane symmetry in the atomic arrangement in the x-axis direction. The information processing device 100 specifies, for example, one or more atoms at least a part of which is included in the selected space 711, other than the atoms included in one part 712, 715 of each detected pair.

Here, it is considered equivalent to determine the plane-symmetrical positions in each of the parts 712 and 713 as the adsorption positions of the adsorbate. Therefore, the information processing device 100 may narrow down the range in which the adsorption positions of the adsorbate are searched by excluding the atoms included in one of the parts 712 and 715 of each pair. Furthermore, the information processing device 100 specifies the upper stage of the catalyst represented by the three-dimensional model 700 as the range in which the adsorbate may be adsorbed, taking into account the step structure.

Therefore, in the example of Fig. 7, the candidates for the adsorption positions of the adsorbate related to one or more specified atoms in the range in which the adsorbate can be adsorbed are the candidates indicated by the hatches with diagonal lines slanting downward to the right. As a result, if the information processing device 100 considers the candidates indicated by the hatches with diagonal lines slanting downward to the right based on the plane symmetry, it may substantially consider the candidates indicated by the dotted hatches. Therefore, the information processing device 100 may narrow down the range in which the adsorption positions of the adsorbate are searched.

(7-3) The information processing device 100 determines the adsorption position of the adsorbent from among one or more specified atoms for the three-dimensional model 700. For example, the information processing device 100 randomly determines the adsorption position of the adsorbent from among candidates for the adsorption position of the adsorbent formed by one or more specified atoms for the three-dimensional model 700.

In the example of Fig. 7, the information processing device 100 for example determines an adsorption position 703 of the adsorbent. The information processing device 100 stores the adsorption of the adsorbent to the determined adsorption position 703 of the adsorbent in association with the three-dimensional model 700. Here, in order to improve the accuracy of analyzing the characteristics, etc. of the catalyst, etc., it may be preferable for the information processing device 100 to randomly determine the adsorption position of the adsorbent in order to reduce the artificiality of the adsorption position of the adsorbent.

As a result, the information processing device 100 may determine the adsorption position of the adsorbent in the three-dimensional model 700, taking into account the overlap of the adsorption positions of the adsorbent. The information processing device 100 may reduce the range for searching for the adsorption position of the adsorbate to about 10/16. Therefore, the information processing device 100 may specify how the adsorbate is adsorbed to the catalyst in the simulation. The information processing device 100 may generate a combination of the three-dimensional model 700 and the adsorption position of the adsorbate that is suitable for the simulation.

Here, a case has been described in which the information processing device 100 randomly determines the adsorption position of the adsorbate from among candidates for the adsorption position of the adsorbate formed by one or more specified atoms for the three-dimensional model 700, but the present invention is not limited thereto. For example, the information processing device 100 may comprehensively determine the adsorption position of the adsorbent from among candidates of the adsorption position of the adsorbent formed by one or more specified atoms for the three-dimensional model 700.

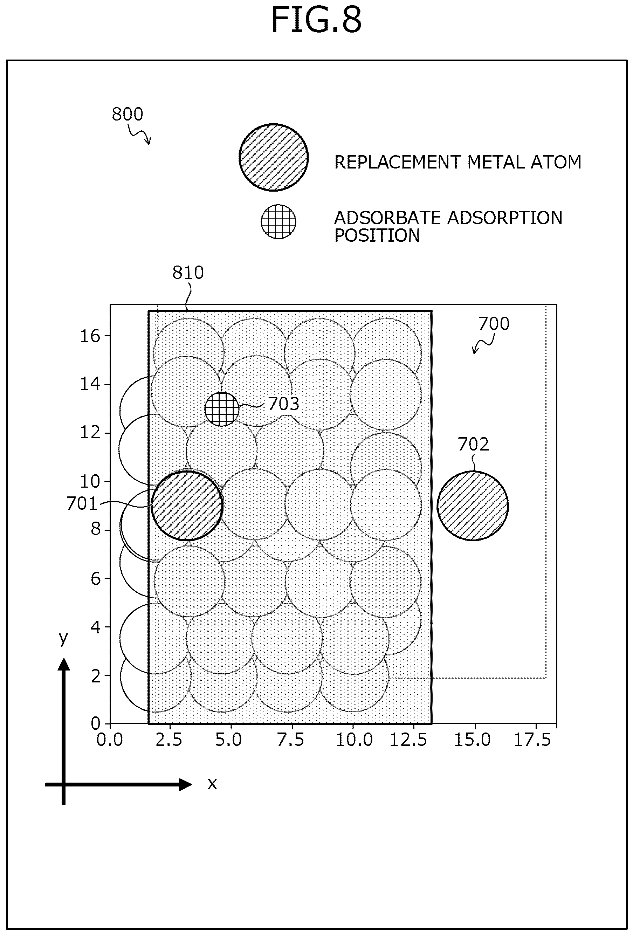

The information processing device 100 may prepare M combinations of the three-dimensional model 700 and the adsorption position of the adsorbent by associating the adsorption position of the adsorbent with the three-dimensional model 700 for each determined adsorption position of the adsorbent. When the information processing device 100 prepares N three-dimensional models 700, it prepares N*M combinations of the three-dimensional model 700 and the adsorption position of the adsorbent. In this case, the information processing device 100 executes the following process for each prepared combination. Next, we move to the explanation of Fig. 8.

In Fig. 8, a graph 800 depicts the three-dimensional model 700 from the z-axis direction. In the three-dimensional model 700, there are atoms 701 of the first replacement metal indicated by hatching with diagonal lines rising to the right. According to the periodic boundary condition, the information processing device 100 may consider the presence of the first replacement metal atom 702 indicated by the hatch with diagonal lines rising to the right due to the presence of the first replacement metal atom 701 in the three-dimensional model 700. The three-dimensional model 700 is associated with, for example, the adsorption position of the adsorbate indicated by the cross hatch.

(8-1) The information processing device 100 determines that the three-dimensional model 700 associated with the adsorption position of the adsorbate is not a repetition of a space in which a certain pattern of atomic arrangement including the adsorption position of the adsorbate appears in the x-axis direction. For this reason, the information processing device 100 selects an entire space 810 of the three-dimensional model 700 as a repetition unit.

(8-2) The information processing device 100 specifies one or more atoms at least a part of which is included in the selected space 810, and specifies a range in which to search for the base metal atom to be replaced with the atom of the second replacement metal. For example, in the selected space 810, since there are no two parts having plane symmetry in the arrangement of atoms including the adsorption position of the adsorbent in the x-axis direction, the information processing device 100 identifies one or more atoms at least a part of which is included in the selected space 810.

(8-3) The information processing device 100 determines an atom of the base metal to replace an atom of the second replacement metal from among the one or more identified atoms for the three-dimensional model 700. For example, the information processing device 100 randomly determines an atom of the base metal to replace an atom of the second replacement metal from among the one or more identified atoms for the three-dimensional model 700.

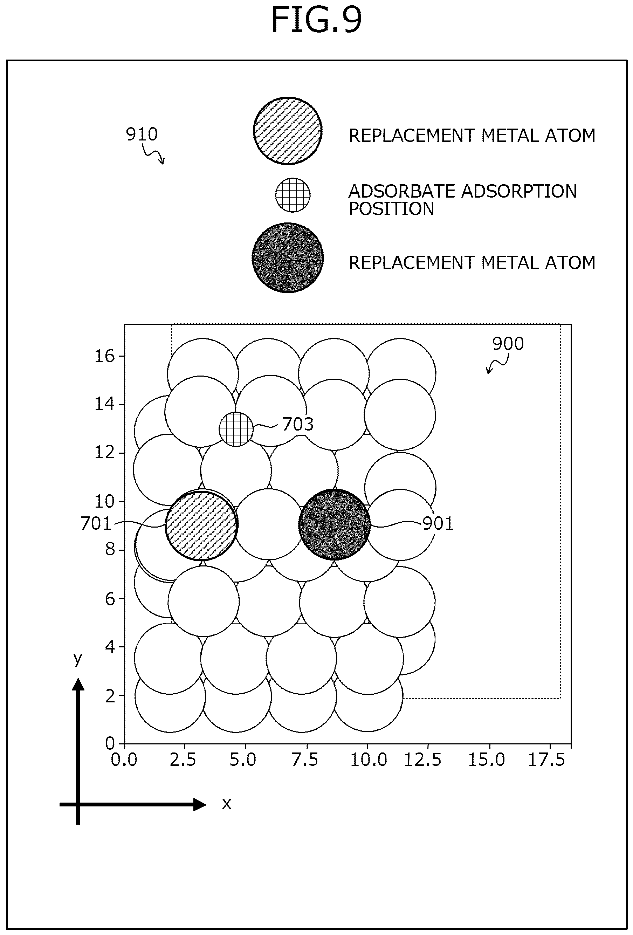

The information processing device 100 updates the three-dimensional model 700 to a three-dimensional model 900 described later in Fig. 9 so that the determined atom of the base metal replaces an atom of the second replacement metal. Here, in order to improve the accuracy of analyzing the characteristics, etc. of the catalyst, etc., and to reduce the artificiality of the updated three-dimensional model 900, it may be preferable for the information processing device 100 to randomly determine the atoms of the base metal to be replaced by the atoms of the second replacement metal.

Here, the case where the information processing device 100 determines the atoms of the base metal to be replaced by the atoms of the second replacement metal after considering the repetition of space and the plane symmetry has been described, but this is not the only case. For example, the information processing device 100 may determine the atoms of the base metal to be replaced by the atoms of the second replacement metal without considering the repetition of space and the plane symmetry.

For example, in the three-dimensional model 700 after the atoms of the base metal are replaced by the atoms of the first replacement metal and the adsorption positions of the adsorbent are associated, the repetition of space tends to be difficult to occur. Similarly, for example, in the three-dimensional model 700 after the atoms of the base metal are replaced by the atoms of the first replacement metal and the adsorption positions of the adsorbent are associated, the plane symmetry tends to be difficult to occur.

Hence, the information processing device 100 may replace the atoms of the base metal with the atoms of the first replacement metal, and after associating the adsorption positions of the adsorbate, determine the atoms of the base metal to be replaced with the atoms of the second replacement metal without considering spatial repetition and plane symmetry. This allows the information processing device 100 to reduce the processing load. Next, we move on to the explanation of Fig. 9.

In Fig. 9, a graph 910 depicts the updated three-dimensional model 900 from the z-axis direction. In the three-dimensional model 900, there are atoms 901 of the second replacement metal indicated by black circles. This allows the information processing device 100 to update the updated three-dimensional model 900 so that it represents a catalyst suitable for a simulation that includes atoms of different metals. The information processing device 100 may generate a combination of the updated three-dimensional model 900 representing a catalyst suitable for a simulation and the adsorption position of the adsorbate.

Here, the case has been described where the information processing device 100 randomly determines, from among one or more specified atoms for the three-dimensional model 600, an atom of the base metal to be replaced with an atom of the second replacement metal, but this is not limited thereto. For example, the information processing device 100 may comprehensively determine, from among one or more specified atoms for the three-dimensional model 600, an atom of the base metal to be replaced with an atom of the second replacement metal.

The information processing device 100 may update the three-dimensional model 700 so that, for each determined atom of the base metal, the atom is replaced with an atom of the second replacement metal, thereby preparing L updated three-dimensional models 900. L is the number of atoms specified, which is one or more. In this case, the information processing device 100 will execute the following process for each updated three-dimensional model 900.

When the information processing device 100 prepares N*M combinations in Fig. 7, it prepares N*M*L combinations of the three-dimensional model 900 and the adsorption position of the adsorbent in Fig. 9. In this case, the information processing device 100 executes the following process for each prepared combination.

The information processing device 100 may prepare a plurality of combinations by repeatedly carrying out a series of processes depicted in Figs. 6 to 9, which prepare one combination of the three-dimensional model 900 and the adsorption position of the adsorbent. As described, the information processing device 100 may make the prepared combinations more likely to have randomness, and may make the prepared combinations less likely to have intentionality.

The information processing device 100 employs, as simulation data, a combination of the updated three-dimensional model 900, which represents a catalyst suitable for the simulation, and the adsorption position of the adsorbent on the catalyst. Based on the adopted simulation data, the information processing device 100 simulates the adsorption of the adsorbate to a catalyst of an alloy containing atoms of different metals, and performs a simulation regarding the reaction or action of the adsorbate on the catalyst.

This allows the information processing device 100 to analyze the properties of the catalyst. As described above, the information processing device 100 may narrow down the range of searching for the base metal atom to replace the atom of the first replacement metal, the adsorption position of the adsorbate, and the base metal atom to replace the atom of the second replacement metal.

Accordingly, the information processing device 100 may prepare only simulation data that is suitable for simulation and is suitable for the use of analyzing the properties of the catalyst. Therefore, the information processing device 100 may reduce the processing time required to analyze the properties of the catalyst, and may improve the accuracy of analyzing the properties of the catalyst.

Here, the case where the information processing device 100 replaces one atom of the base metal with an atom of the first replacement metal and replaces one atom of the base metal with an atom of the second replacement metal has been described, but the present invention is not limited thereto. For example, the information processing device 100 may store a preset ratio of the base metal atom, the first replacement metal atom, and the second replacement metal atom for the catalyst. In this case, the information processing device 100 may replace two or more of the base metal atoms with the first replacement metal atom according to the stored ratio. The information processing device 100 may replace two or more of the base metal atoms with the second replacement metal atom according to the stored ratio.

Here, the case where the information processing device 100 determines the base metal atom to be replaced with the first replacement metal atom and then determines the adsorption position of the adsorbent has been described, but this is not limited thereto. For example, the information processing device 100 may determine the base metal atom to be replaced with the first replacement metal atom and then determines the adsorption position of the adsorbent.

Here, the case where the information processing device 100 determines the base metal atom to be replaced with the second replacement metal atom and then determines the adsorption position of the adsorbent has been described, but this is not limited thereto. For example, the information processing device 100 may determine the atom of the base metal to be replaced by the atom of the first replacement metal, and may further determine the atom of the base metal to be replaced by the atom of the second replacement metal, and then may determine the adsorption position of the adsorbent.

Here, the case where there are two replacement metals has been described, but the present invention is not limited thereto. For example, there may be a case where there is only one replacement metal. In this case, the information processing device 100 adopts a combination of the three-dimensional model 700 generated in the same manner as in Fig. 7 and the adsorption position of the adsorbent as simulation data. For example, there may be three or more replacement metals. In this case, the information processing device 100 determines the atom of the base metal to be replaced by the atom of the third or subsequent replacement metal, as in Fig. 8.

Here, the case where there is one adsorbent has been described, but the present invention is not limited thereto. For example, there may be a case where there are plural adsorbents. For example, there may be a case where there are plural adsorbents of the same type. For example, there may be a case where there are plural adsorbents of different types.

As a result, the information processing device 100 may process the three-dimensional model 101 to represent a sample of the state of the alloy catalyst suitable for the simulation. The information processing device 100 may easily prepare a sample of the state of the alloy catalyst suitable for the simulation. The information processing device 100 may generate simulation data in which a sample of the state of the alloy catalyst suitable for the simulation is associated with the adsorption position of the adsorbate.

Hence, the information processing device 100 may easily analyze the characteristics, etc. of the catalyst. The information processing device 100 may reduce the processing time required to analyze the characteristics, etc. of the catalyst. The information processing device 100 may improve the accuracy of analyzing the characteristics, etc. of the catalyst.

The information processing device 100 may reduce the number of simulation data used in the simulation, for example, and may reduce the processing time required to analyze the characteristics, etc. of the catalyst. In the examples of Figures 6 to 9, the information processing device 100 may for example reduce the number of simulation data by about 8.4% compared to the case where the spatial repetition and plane symmetry are not considered.

The information processing device 100 may , for example, easily prepare simulation data to be used in a simulation without duplication, and may easily analyze the characteristics, etc. of a catalyst with high accuracy. The information processing device 100 may, for example, reduce the artificiality that appears in the simulation data to be used in a simulation, and may easily analyze the characteristics, etc. of a catalyst with high accuracy.

The information processing device 100 is applied, for example, to the chemical field. For example, the information processing device 100 is considered to be utilized by researchers in charge of basic research on catalysts, or researchers in charge of product development using catalysts, in the chemical field.

### (Overall Processing Procedure)

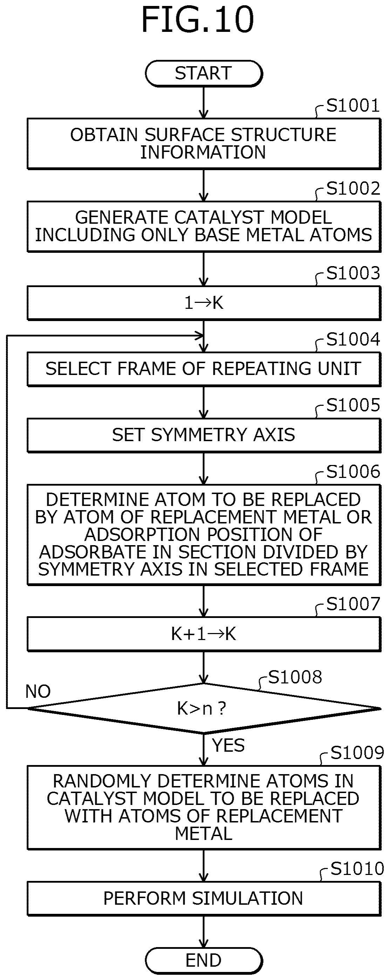

An example of an overall processing procedure executed by the information processing device 100 will then be described with reference to Fig. 10. The overall processing is realized, for example, by the CPU 301 depicted in Fig. 3, storage areas such as the memory 302 and the recording medium 305, and the network I/F 303.

Fig. 10 is a flowchart depicting an example of an overall processing procedure. 10, the information processing device 100 obtains information on the surface structure of the catalyst (step S1001). The information processing device 100 generates a catalyst model including only base metal atoms (step S1002). The catalyst model represents the arrangement of atoms in the catalyst.

The information processing device 100 sets a variable K to 1 (step

S1003). The information processing device 100 identifies a repeating unit in the catalyst model and selects a frame of the repeating unit (step S1004). The information processing device 100 sets a symmetry axis in the selected frame (step S1005).

The information processing device 100 determines an atom to be replaced by an atom of the replacement metal or an adsorption position of the adsorbate in a section divided by the symmetry axis in the selected frame (step S1006). The information processing device 100 determines an atom to be replaced by an atom of the replacement metal or an adsorption position of the adsorbate from a range of atoms at least partially included in the section divided by the symmetry axis in the selected frame.

The information processing device 100 for example determines an atom to be replaced by an atom of the replacement metal when K=1. The information processing device 100 for example determines an adsorption position of the adsorbate when K=2. The information processing device 100 for example determines an atom to be replaced by an atom of the replacement metal when K≥3. The information processing device 100 increments the variable K (step S1007).

The information processing device 100 judges whether K>n (step S1008). If K>n (step S1008: YES), the information processing device 100 proceeds to the process of step S1009. On the other hand, if K>n but K≤n (step S1008: NO), the information processing device 100 returns to the process of step S1004.

At step S1009, the information processing device 100 randomly determines atoms in the catalyst model to be replaced with atoms of the replacement metal (step S1009). The information processing device 100 performs a simulation based on the catalyst model (step S1010). The information processing device 100 ends the entire process.

This allows the information processing device 100 to determine a combination of the catalyst model and the adsorption position of the adsorbate that is suitable for the simulation. The information processing device 100 may perform a simulation with high accuracy. The information processing device 100 may execute the overall process depicted in Fig. 10 plural times. As a result, the information processing device 100 may perform a simulation for various combinations, and may analyze the properties of the catalyst efficiently and accurately.

Here, the information processing device 100 may execute some steps of the process in Fig. 10 in a different order. The information processing device 100 may also omit some steps of the process in Fig. 10. For example, the process of step S1010 may be omitted.

As set forth hereinabove, the information processing device 100 may obtain a three-dimensional model that represents the arrangement of a plurality of atoms that form a catalyst containing atoms of a certain metal. According to the information processing device 100, when the obtained three-dimensional model is a repetition of spaces in which an atomic arrangement of a certain pattern appears in a first direction, it is possible to select any space in the obtained three-dimensional model in which the atomic arrangement of the certain pattern appears. According to the information processing device 100, it is possible to identify one or more atoms that are at least partially included in any of the selected spaces. According to the information processing device 100, it is possible to determine, for the obtained three-dimensional model, an atom to be replaced with an atom of another metal different from a certain metal, or an adsorption position of an adsorbent, from among one or more specified atoms. This allows the information processing device 100 to easily generate a combination of a three-dimensional model and an adsorption position of an adsorbent that is suitable for a simulation. Therefore, the information processing device 100 may reduce the processing time required for analyzing the characteristics, etc. of a catalyst.

According to the information processing device 100, it is possible to determine whether two parts having symmetry in the arrangement of atoms exist in a first direction in any selected space. If they exist, according to the information processing device 100, it is possible to specify one or more atoms of the obtained three-dimensional model that are at least partially included in any selected space, other than the atoms included in one of the two parts. This allows the information processing device 100 to narrow down the range in which to search for an atom to be replaced with an atom of another metal different from a certain metal, or an adsorption position of an adsorbent. Therefore, the information processing device 100 may easily generate a combination of a three-dimensional model and an adsorption position of an adsorbent that is suitable for a simulation. The information processing device 100 may reduce the processing time required for analyzing the characteristics, etc. of a catalyst.

According to the information processing device 100, it is possible to determine whether two parts having symmetry in the atomic arrangement exist in the first direction in any of the selected spaces. According to the information processing device 100, if there are no symmetry in the atomic arrangement, it is possible to specify all atoms of the obtained three-dimensional model that are at least partially included in any of the selected spaces. As a result, when there are no two parts having symmetry in the atomic arrangement, the information processing device 100 may appropriately set an atom to be replaced with an atom of a metal different from the certain metal, or a range for searching for an adsorption position of an adsorbate.

According to the information processing device 100, when any atom to be replaced with an atom of a metal different from the certain metal is determined from among one or more specified atoms, any atom in the three-dimensional model may be replaced with an atom of a metal different from the certain metal. According to the information processing device 100, when the three-dimensional model after the replacement is a repetition of spaces in which a certain pattern of atomic arrangement appears in a first direction, it is possible to select any of the spaces in which the certain pattern of atomic arrangement appears in the three-dimensional model after the replacement. According to the information processing device 100, it is possible to determine whether two parts having symmetry in the atomic arrangement exist in the first direction in any of the selected spaces. According to the information processing device 100, if they exist, it is possible to specify one or more atoms that are at least partially included in any of the selected spaces other than the atoms included in one of the two parts in the three-dimensional model after the replacement. According to the information processing device 100, it is possible to determine, from among the one or more specified atoms, an atom to be replaced with an atom of a metal other than a certain metal, or an adsorption position of an adsorbent, for the three-dimensional model after the replacement. Thereby, the information processing device 100 may repeatedly determine any of the atoms to be replaced with an atom of a metal other than a certain metal. After determining any of the atoms to be replaced with an atom of a metal other than a certain metal, the information processing device 100 may further determine the adsorption position of an adsorbent.

According to the information processing device 100, when the obtained three-dimensional model is not a repetition of a space in which a certain pattern of atomic arrangement appears in the first direction, the entire space of the obtained three-dimensional model may be selected. As a result, even when the repetition of a space in which a certain pattern of atomic arrangement appears does not appear, the information processing device 100 may appropriately set a range for searching for an atom to be replaced with an atom of another metal different from a certain metal, or an adsorption position of an adsorbate.

According to the information processing device 100, for the obtained three-dimensional model, a contact position in contact with all three atoms adjacent to each other in one or more specified atoms may be determined as an adsorption position of an adsorbate. As a result, the information processing device 100 may appropriately determine an adsorption position of an adsorbate.

According to the information processing device 100, a three-dimensional model that satisfies a periodic boundary condition may be obtained. As a result, the information processing device 100 may determine an atom to be replaced with an atom of another metal different from a certain metal, or an adsorption position of an adsorbate, taking into account the periodic boundary condition.

The information processing method described in this embodiment may be implemented by executing a program prepared in advance on a computer such as a PC or a workstation. The information processing program described in this embodiment is recorded on a computer-readable recording medium and executed by being read from the recording medium by the computer. The recording medium may be a hard disk, a flexible disk, a compact disc (CD)-ROM, a magneto optical disc (MO), a digital versatile disc (DVD), etc. The information processing program described in the present embodiment may be distributed via a network such as the Internet.

### EXPLANATIONS OF LETTERS OR NUMERALS

100 information processing device
101, 500, 600, 700, 900 3-dimensional model
110, 611, 711, 810 space
111, 112, 612, 613, 712~715 parts
120, 140 range
130 symmetry plane
200 information processing system
201 simulation device
202 client device
210 network
300 bus
301 CPU
302 memory
303 network I/F
304 recording medium I/F
305 recording medium
400 storage unit
401 obtaining unit
402 selecting unit
403 identifying unit
404 determining unit
405 updating unit
406 simulating unit
407 output unit
501 catalyst
502 adsorbate
610, 620, 630, 710, 800, 910 graphs
701, 702, 901 atoms
703 adsorption position

## Claims

1. An information processing program for causing a computer to execute a process comprising:
obtaining a three-dimensional model representing an arrangement of a plurality of atoms that form a catalyst containing an atom of a first metal;
when the obtained three-dimensional model is a repetition of spaces in which a certain pattern of atomic arrangement appears in a first direction, selecting from the obtained three-dimensional model, any of the spaces in which the certain pattern of atomic arrangement appears;
identifying one or more atoms at least partially included in the selected any of the spaces; and
determining, from among the identified one or more atoms of the obtained three-dimensional model, a first atom to be replaced with a second atom of a second metal different from the first metal, or an adsorption position of an adsorbate.

2. The information processing program according to claim 1, wherein
when two parts each having symmetry in the atomic arrangement in the first direction are present in the selected any of the spaces, the identifying includes identifying the one or more atoms at least partially included in the selected any of the spaces in the obtained three-dimensional model, exclusive of atoms included in one of the two parts.

3. The information processing program according to claim 2, wherein
when the two parts each having symmetry in the atomic arrangement in the first direction are absent in the selected any of the spaces, the identifying includes identifying all atoms at least partially included in the selected any of the spaces in the obtained three-dimensional model.

4. The information processing program according to claim 2 or 3, the process further comprising:
when the first atom to be replaced with the second atom of the second metal different from the first metal is determined from among the identified one or more atoms, replacing the first atom with the second atom of the second metal different from the first metal in the three-dimensional model, wherein
when the three-dimensional model after the replacing is a repetition of spaces in which the certain pattern of atomic arrangement appears in the first direction, the selecting includes selecting the any of the spaces in which the certain pattern of atomic arrangement appears in the three-dimensional model after the replacing,
when the two parts each having symmetry in the atomic arrangement in the first direction are present in the selected any of the spaces, the identifying includes identifying one or more atoms at least partially included in the selected any of the spaces in the three-dimensional model after the replacing, exclusive of atoms included in one of the two parts, and
the determining includes, for the three-dimensional model after the replacing, determining from among the identified one or more atoms, the first atom to be replaced with the second atom of the second metal different from the first metal, or an adsorption position of an adsorbate.

5. The information processing program according to claim 2 or 3, wherein
when the obtained three-dimensional model is not a repetition of spaces in which the certain pattern of atomic arrangement appears in the first direction, the selecting includes selecting an entire space of the obtained three-dimensional model.

6. The information processing program according to claim 2 or 3, wherein
the determining includes, for the obtained three-dimensional model, determining as the adsorption position of the adsorbate, a contact position that is in contact with all of any three atoms adjacent to each other in the identified one or more atoms.

7. The information processing program according to claim 2 or 3, wherein
the three-dimensional model satisfies a periodic boundary condition.

8. The information processing program according to claim 2 or 3, wherein
when the adsorption position of the adsorbate is already determined, the atomic arrangement includes the adsorption position of the adsorbate.

9. An information processing method executed by a computer, the information processing method comprising:
obtaining a three-dimensional model representing an arrangement of a plurality of atoms that form a catalyst containing an atom of a first metal;
when the obtained three-dimensional model is a repetition of spaces in which a certain pattern of atomic arrangement appears in a first direction, selecting from the obtained three-dimensional model, any of the spaces in which the certain pattern of atomic arrangement appears;
identifying one or more atoms at least partially included in the selected any of the spaces; and
determining, from among the identified one or more atoms of the obtained three-dimensional model, a first atom to be replaced with a second atom of a second metal different from the first metal, or an adsorption position of an adsorbate.

10. An information processing device, comprising a controller configured to:
obtain a three-dimensional model representing an arrangement of a plurality of atoms that form a catalyst containing an atom of a first metal;
when the obtained three-dimensional model is a repetition of spaces in which a certain pattern of atomic arrangement appears in a first direction, select from the obtained three-dimensional model, any of the spaces in which the certain pattern of atomic arrangement appears;
identify one or more atoms at least partially included in the selected any of the spaces; and
determine, from among the identified one or more atoms of the obtained three-dimensional model, a first atom to be replaced with a second atom of a second metal different from the first metal, or an adsorption position of an adsorbate.

11. An information processing program for causing a computer to execute a process comprising:
obtaining a three-dimensional model representing an arrangement of a plurality of atoms that form a catalyst containing a first atom of a first metal;
selecting an entire space of the obtained three-dimensional model;
when two parts having symmetry in an atomic arrangement are present in a first direction in the selected space, identifying one or more atoms at least partially included in the selected space in the obtained three-dimensional model, exclusive of atoms included in one of the two parts; and
determining, from the identified one or more atoms of the obtained three-dimensional model, the first atom to be replaced with a second atom of a second metal different from the first metal, or an adsorption position of an adsorbate.
